# EUROPÄISCHE PATENTSCHRIFT

(11) **EP 3 988 132 B1**
(45) Veröffentlichungstag und Bekanntmachung des Hinweises auf die Patenterteilung: **23.08.2023**
(21) Anmeldenummer: 21202166.1
(22) Anmeldetag: 12.10.2021
(51) Int. Cl.: A61L 2/24, G01C 21/00, G05D 1/00, G05D 1/02, B05B 14/30, B05B 15/68

(54) **SICH SELBSTTÄTIG FORTBEWEGENDER DESINFEKTIONSROBOTER ZUM DESINFIZIEREN VON OBERFLÄCHEN**
SELF-PROPELLED DISINFECTION ROBOT FOR DISINFECTING SURFACES
ROBOT DE DÉSINFECTION AUTONOME PERMETTANT DE DÉSINFECTER DES SURFACES

(30) Priorität: 23.10.2020 DE 102020127988
(43) Veröffentlichungstag der Anmeldung: 27.04.2022
(73) Patentinhaber: Vorwerk & Co. Interholding GmbH, 42275 Wuppertal (DE)
(72) Erfinder: Dr. Hayn, Henning, 40723 Hilden (DE); Mosebach, Andrej, 59425 Unna (DE); Hackert, Georg, 44869 Bochum (DE)
(74) Vertreter: Müller, Enno

(56) Entgegenhaltungen:
- WO-A1-2020/041817
- WO-A2-2018/167760
- CN-A- 111 214 680
- US-A1- 2016 271 803
- US-A1- 2018 074 508
- US-A1- 2019 262 489

## Beschreibung

### Gebiet der Technik

Die Erfindung betrifft einen sich selbsttätig fortbewegenden Desinfektionsroboter zum Desinfizieren von Oberflächen, mit einem Gerätegehäuse, einer Antriebseinrichtung zur Fortbewegung des Desinfektionsroboters innerhalb einer Umgebung mit mindestens einem Raum, einer Desinfektionseinrichtung zum Ausführen einer Desinfektionstätigkeit innerhalb der Umgebung und einer Steuereinrichtung zum Steuern der Desinfektionstätigkeit, wobei die Steuereinrichtung eingerichtet ist, vollautomatisiert eine lokal begrenzte Desinfektion in ausschließlich nur einem dafür vordefinierten Teilbereich des Raumes der Umgebung oder eine lokal begrenzte Desinfektion von ausschließlich nur einem dafür definierten Objekt in dem Raum der Umgebung zu steuern, wobei die Desinfektionseinrichtung zum Erreichen einer lokal begrenzten Desinfektionswirkung eine Abschirmeinrichtung, nämlich Einhausung, zum Separieren des zu desinfizierenden, lokal begrenzten Teilbereiches von benachbarten Teilbereichen desselben Raumes aufweist, wobei die Abschirmeinrichtung ein lediglich einseitig offenes Gehäuse ist, welches um das zu desinfizierende Objekt oder an die zu desinfizierende Oberfläche gelegt wird, so dass die Desinfektion lediglich innerhalb des Gehäuses stattfindet, jedoch nicht außerhalb des Gehäuses.

### Stand der Technik

Desinfektionsroboter sind im Stand der Technik beispielsweise zur Desinfektion von Supermärkten und Lagerhäusern oder auch zur Desinfektion von Zimmern in Krankenhäusern bekannt. Die Desinfektionsroboter nutzen UV-Strahlung, insbesondere UV-B oder UV-C-Strahlung, welche von Leuchtelementen in die Umgebung emittiert wird. Einen solchen Roboter offenbart beispielsweise die US 2016/0271803 A1.

Daneben sind, beispielsweise aus der US 2019/0262489 A1, Systeme bekannt, welche mittels eines Plasmas sterilisieren. Dies auch unter Nutzung einer Einhausung zum Schutz der Umwelt.

Die CN 111214680 A und WO 2018/167760 A2 offenbaren des Weiteren Fluggeräte zur Detektion und Elimination schädlicher Substanzen.

Nachteilig ist dabei, dass in der Umgebung des Desinfektionsroboters keine Personen oder Tiere anwesend sein dürfen, da die UV-Strahlung ungerichtet in die Umgebung emittiert wird und beispielsweise eine Gefahr für Augen und Haut darstellt.

### Zusammenfassung der Erfindung

Ausgehend von dem vorgenannten Stand der Technik ist es Aufgabe der Erfindung, einen sich selbsttätig fortbewegenden Desinfektionsroboter zu schaffen, welcher Innenräume, insbesondere Büroräume oder Sanitärräume, desinfizieren kann, ohne die Gesundheit anwesender Personen oder Tiere zu beeinträchtigen.

Zur Lösung dieser Aufgabe wird vorgeschlagen, dass der Desinfektionsroboter eine Navigationseinrichtung zur Navigation und Selbstlokalisierung des Desinfektionsroboters innerhalb der Umgebung anhand einer Umgebungskarte aufweist, und wobei die Steuereinrichtung eingerichtet ist, auf einen Desinfektionsplan zuzugreifen, welcher in einem Speicher des Desinfektionsroboters oder einem in Datenkommunikation mit dem Desinfektionsroboter stehenden externen Speicher gespeichert ist, wobei der Desinfektionsplan eine Vorgabe über eine Desinfektionsleistung, welche die Desinfektionswirkung festlegt, während einer Desinfektionstätigkeit aufweist, wobei stärker kontaminiert Teilbereiche oder Objekte der Umgebung stärker desinfiziert werden als demgegenüber lediglich leicht kontaminierte Objekte oder Teilbereiche, und wobei der Desinfektionsroboter eine Detektionseinrichtung aufweist, welche eingerichtet ist, Lebewesen in der Umgebung zu erkennen, wobei die Steuereinrichtung eingerichtet ist, eine Desinfektionsleistung zu reduzieren und/oder ein Warnsignal auszugeben, wenn die Detektionseinrichtung eine Anwesenheit eines Lebewesens in der Umgebung detektiert.

Erfindungsgemäß können durch den Desinfektionsroboter einzelne Teilbereiche oder Objekte innerhalb eines Raumes, beispielsweise eines Büroraumes, Sanitärraumes oder Raumes eines Privathaushaltes, gezielt abgegrenzt und desinfiziert werden. Die Desinfektion findet vollautomatisiert durch den sich selbsttätig fortbewegenden Desinfektionsroboter statt, so dass kein manuelles Reinigungspersonal erforderlich ist, um kritische Bereiche, wie beispielsweise Türklinken, Toiletten oder Waschbecken, zu desinfizieren. Durch die lokal begrenzte Desinfektion innerhalb des Raumes besteht keine Gesundheitsgefahr für Menschen oder Tiere, die innerhalb desselben Raumes, jedoch außerhalb des für die Desinfektion vordefinierten Teilbereiches des Raumes bzw. lokal begrenzten Objektes anwesend sind. Die zu desinfizierenden Orte, nämlich Teilbereiche eines Raumes oder Objekte, können zum Beispiel Türgriffe, Türklinken, Stühle, Möbelgriffe, Möbeloberflächen, Sanitärobjekte, Fußbodenteilbereiche oder andere üblicherweise mit einem Menschen oder Tier in Kontakt gelangende Oberflächen der Umgebung sein. Der Desinfektionsroboter weist zur Fortbewegung in der Umgebung eine Navigationseinrichtung auf, welche zur Navigation und Selbstlokalisierung in der Umgebung geeignet ist. Die Navigationseinrichtung verfügt des Weiteren üblicherweise über eine Steuerungseinheit, beispielsweise eine Hauptplatine, ggfs. inklusive eines Funkmoduls zur Datenübertragung an eine Antriebseinrichtung des Desinfektionsroboters, welche dem Antrieb von Rädern des Desinfektionsroboters dient. Die Navigationseinrichtung verfügt des Weiteren üblicherweise über einen oder mehrere Sensoren zur Detektion von Hindernissen in der Umgebung. Beispielsweise kann der Sensor ein Abstandssensor sein, welcher Abstände zu Hindernissen misst. Anhand der gemessenen Abstandswerte kann dann eine Umgebungskarte erstellt werden, welche die Umgebung mit darin befindlichen Räumen, Teilbereichen und Objekten wiedergibt, insbesondere in der Form eines Grundrisses mit darin verzeichneten Teilbereichen und Objekten.

Die Desinfektionseinrichtung weist zum Erreichen einer lokal begrenzten Desinfektionswirkung eine Abschirmeinrichtung, nämlich eine Einhausung, zum Separieren des zu desinfizierenden, lokal begrenzten Teilbereiches von benachbarten Teilbereichen desselben Raumes auf. Diese Abschirmeinrichtung ist ein lediglich einseitig offenes Gehäuse, welches um das zu desinfizierende Objekt oder an die zu desinfizierende Fläche gelegt wird, so dass die Desinfektion lediglich innerhalb des Gehäuses stattfindet, jedoch nicht außerhalb des Gehäuses. Lebewesen, die sich außerhalb des Gehäuses befinden, werden durch die Desinfektion nicht beeinträchtigt, insbesondere nicht gesundheitlich geschädigt. Bevorzugt ist, dass eine geöffnete Seite der Abschirmeinrichtung, insbesondere der Einhausung, gegen eine Fläche gelegt und somit verschlossen wird, beispielsweise eine Bodenfläche oder Wandfläche, welche ein zu desinfizierendes Objekt trägt. Die Abschirmeinrichtung, insbesondere Einhausung, kann vorzugsweise größenveränderlich sein, beispielsweise durch relativ zueinander bewegliche Wandungen, so dass die Größe der Abschirmeinrichtung an die Größe eines zu desinfizierenden Objektes oder Teilbereiches angepasst werden kann. Bevorzugt erfolgt diese Größenanpassung vollautomatisch mittels der Steuereinrichtung des Desinfektionsroboters. Bevorzugt erkennt eine Detektionseinrichtung des Desinfektionsroboters eine Größe der zu desinfizierenden Objekte bzw. Teilbereiche des Raumes und passt die Größe der Abschirmeinrichtung entsprechend an.

Es wird des Weiteren vorgeschlagen, dass der zu desinfizierende Teilbereich der Umgebung ein in der Umgebungskarte gespeicherter Ortspunkt, eine in der Umgebungskarte gespeicherte Teilfläche oder ein in der Umgebungskarte gespeichertes Teilvolumen ist. Der zu desinfizierende Teilbereich kann somit eindimensional, zweidimensional oder dreidimensional sein. Ein Teilvolumen kann dabei beispielsweise ein zu desinfizierendes Objekt beinhalten. Bei einem zweidimensionalen Teilbereich handelt es sich beispielsweise um einen Flächenteilbereich eines Fußbodens, einer Wand, einer Raumdecke oder ähnlichem.

Des Weiteren wird vorgeschlagen, dass der Desinfektionsroboter eine Kommunikationsschnittstelle zum Vordefinieren des zu desinfizierenden Teilbereiches oder des zu desinfizierenden Objektes durch einen Nutzer oder Hersteller des Desinfektionsroboters aufweist, und/oder dass der Desinfektionsroboter eine Detektionseinrichtung zum automatischen Erkennen des zu desinfizierenden Teilbereiches oder des zu desinfizierenden Objektes in der Umgebung aufweist. Insbesondere kann auch vorgesehen sein, dass der Teilbereich bzw. das Objekt automatisch von dem Desinfektionsroboter erkannt und zunächst an einen Nutzer des Desinfektionsroboters gemeldet wird, woraufhin der Nutzer dann entscheiden kann, ob der Teilbereich bzw. das Objekt als zu desinfizierend in der Umgebungskarte gespeichert werden soll. Grundsätzlich wird jedoch vorgeschlagen, dass die zu desinfizierenden Orte der Umgebung entweder manuell von einem Nutzer in der erstellten Umgebungskarte markiert, das heißt definiert werden, beispielsweise über eine auf einem Nutzerendgerät installierte Applikation, oder automatisch durch den Desinfektionsroboter identifiziert werden, beispielsweise mittels Objekterkennung. Zur Verwirklichung einer automatischen Erkennung eines zu desinfizierenden Objektes kann der Desinfektionsroboter beispielsweise einen Speicher aufweisen oder auf einen externen Speicher zugreifen, welcher Angaben zu definierten Objekten enthält, welche grundsätzlich zu desinfizieren sind. Bei derartigen vordefinierten Objekten kann es sich beispielsweise um Klinken oder Griffe an Türen oder Möbeln handeln. Durch einen Merkmalsvergleich, beispielsweise im Rahmen einer Bildverarbeitung, kann eine Detektionseinrichtung des Desinfektionsroboters dann erkennen, ob es sich bei einem Objekt oder Teilbereich innerhalb einer Umgebung um ein zu desinfizierendes Objekt oder einen zu desinfizierenden Teilbereich handelt.

Es kann vorgesehen sein, dass die Steuereinrichtung eingerichtet ist, auf einen Desinfektionsplan zuzugreifen, welcher in einem Speicher des Desinfektionsroboters oder einem in Datenkommunikation mit dem Desinfektionsroboter stehenden externen Speicher gespeichert ist, wobei der Desinfektionsplan eine Vorgabe über eine Reihenfolge von mehreren auszuführenden Desinfektionstätigkeiten, eine Vorgabe über eine Wiederholungsfrequenz von Desinfektionstätigkeiten, eine Vorgabe über eine Desinfektionsleistung während einer Desinfektionstätigkeit und/oder eine Vorgabe über einen Desinfektionszeitpunkt einer Desinfektionstätigkeit aufweist. Gemäß einer Ausführung kann die Steuereinrichtung des Desinfektionsroboters aus dem Speicher eine Abfolge der zu desinfizierenden Orte der Umgebung abrufen, oder auch selbst festlegen. Sofern die Steuereinrichtung eingerichtet ist, die Reihenfolge der Desinfektion an mehreren Orten selbst festzulegen, kann eine Planungsvorgabe beispielsweise eine detektierte Kontaminationsstärke von an einem Ort vorhandenen Viren, Bakterien, Pilzen und dergleichen, eine Entfernung des Desinfektionsroboters zu den jeweiligen Orten, eine vorteilhafte Einbindung des Ortes in einen geplanten Desinfektionspfad durch die Umgebung, oder ähnliches sein. Die Steuereinrichtung steuert die Antriebseinrichtung des Desinfektionsroboters anschließend zu den vorgeplanten Orten der Umgebung, an welchen dann eine vollautomatische Desinfektion stattfinden kann. Nach der Desinfektion kann die Desinfektionstätigkeit in einem Protokoll dokumentiert werden, insbesondere als erfolgreich abgeschlossen gespeichert werden. Beispielsweise können die erfolgreich desinfizierten Orte graphisch in der Umgebungskarte markiert werden, so dass ein Nutzer besonders anschaulich Kenntnis über den Desinfektionsablauf und -erfolg erhält. In diesem Sinne kann ein Report über die Desinfektionstätigkeiten erstellt werden. Ein gespeicherter Desinfektionsplan kann zudem eine Vorgabe über einen Zeitpunkt oder eine Wiederholungsfrequenz von Desinfektionstätigkeiten aufweisen. Beispielsweise kann eine Desinfektion eines bestimmten Teilbereiches oder Objektes oder auch eine Abfolge mehrerer Desinfektionstätigkeiten stets zu einem bestimmten Zeitpunkt gestartet werden, beispielsweise täglich abends um 20 Uhr oder ähnliches. Alternativ können auch unregelmäßig wiederkehrende Reinigungstätigkeiten definiert sein. Erfindungsgemäß ist eine Desinfektionsleistung gespeichert, welche die Desinfektionswirkung festlegt. Stärker kontaminierte Teilbereiche oder Objekte der Umgebung werden somit stärker desinfiziert als demgegenüber lediglich leicht kontaminierte Objekte oder Teilbereiche.

Der Desinfektionsplan kann durch einen Nutzer des Desinfektionsroboters variierbar sein, insbesondere mit Hilfe einer auf einem externen Nutzerendgerät gespeicherten Applikation variiert werden, welche eingerichtet ist, auf den Speicher zuzugreifen. Desinfektionstätigkeiten können somit nach Wahl und Belieben des Nutzers festgelegt werden. Der Nutzer kann dafür ein Nutzerendgerät verwenden und muss die Eingaben nicht an dem Desinfektionsroboter selbst vornehmen. Auf diese Art und Weise kann er besonders bequem Eingaben an einem Mobiltelefon, Laptop, Tablet oder ähnlichem vornehmen.

Des Weiteren kann vorgehen sein, dass die Desinfektionseinrichtung eine UV-Lichtquelle, insbesondere mindestens eine UV-LED, eine Plasmaquelle und/oder eine Sprüheinrichtung zum Versprühen einer Desinfektionslösung aufweist. Bei der UV-Lichtquelle kann es sich insbesondere um eine Lichtquelle handeln, welche UV-Licht in einem UV-C oder UV-B-Spektrum emittiert, das heißt in einem Wellenlängenbereich von 100 Nanometer bis 280 Nanometer (LTVC) oder 280 Nanometer bis 315 Nanometer (UVB). Diese Wellenlängen sind insbesondere geeignet, um Viren, Bakterien und Pilze zu entfernen bzw. unschädlich zu machen. Alternativ zu UV-Lichtquellen kann auch eine Plasmaquelle eingesetzt werden, die freie Ladungsträger, insbesondere Ionen und Elektronen, emittiert. Diese freien Ladungsträger sind vorzugsweise ebenfalls geeignet, um Viren, Bakterien und Pilze unschädlich zu machen. Eine weitere Ausführung kann vorsehen, dass die Desinfektionseinrichtung über eine Sprüheinrichtung verfügt, die eine Desinfektionslösung auf einen zu desinfizierenden Teilbereich oder ein Objekt sprühen kann. Auch diesbezüglich ist die Sprühquelle insbesondere durch eine Einhausung von benachbarten Teilbereichen oder Teilvolumina des Raumes abgeschirmt, so dass in der Umgebung anwesende Lebewesen nicht mit der Desinfektionslösung in Kontakt gelangen.

Besonders bevorzugt weist der Desinfektionsroboter eine Verlagerungseinrichtung zur Verlagerung der Desinfektionseinrichtung relativ zu dem Gerätegehäuse auf. Insbesondere kann die Verlagerungseinrichtung ausgebildet sein, eine Höhenveränderung der Desinfektionseinrichtung hervorzurufen. Dadurch kann die Desinfektionseinrichtung, bevorzugt inklusive der Abschirmeinrichtung, beispielsweise an verschiedene Höhen eines zu desinfizierenden Teilbereiches oder Objektes angepasst werden. Diese Ausführung eignet sich insbesondere, wenn beispielsweise Griffe, Klinken oder ähnliches, welche sich in einem Überbodenbereich befinden, desinfiziert werden sollen.

Die Verlagerungseinrichtung wird vorzugsweise von der Steuereinrichtung gesteuert, welche Kenntnis über die Höhe von zu desinfizierenden Objekten bzw. Teilbereichen hat. Die Höhe kann durch die Detektionseinrichtung des Desinfektionsroboters ermittelt werden. Gemäß einer Ausführung kann die Verlagerungseinrichtung des Desinfektionsroboters auch ausgebildet sein, die Desinfektionseinrichtung in verschiedene Winkelstellungen zu verlagern, so dass Objekte oder zu desinfizierende Teilbereiche aus verschiedenen Richtungen desinfiziert werden können.

Gemäß einer weiteren Ausführung kann der Desinfektionsroboter ein Bodenfahrzeug oder eine Flugdrohne sein. Sofern es sich um eine Flugdrohne handelt, kann diese zu den zu desinfizierenden Teilbereichen bzw. Objekten hinfliegen und diese desinfizieren, insbesondere auch mittels der Abschirmeinrichtung von benachbarten Teilbereichen des Raumes abgrenzen. Bevorzugt eignet sich eine Flugdrohne auch, um eine Desinfektion des Teilbereiches bzw. des Objektes von oben vorzunehmen, insbesondere auch, um eine Sprühlösung von oben aufzusprühen.

Erfindungsgemäß ist vorgesehen, dass der Desinfektionsroboter eine Detektionseinrichtung aufweist, welche eingerichtet ist, Lebewesen, insbesondere Menschen und Tiere, in der Umgebung zu erkennen. Die Steuereinrichtung des Desinfektionsroboters kann eingerichtet sein, eine Desinfektionstätigkeit zeitlich aufzuschieben und/oder eine Desinfektionstätigkeit abzubrechen, wenn die Detektionseinrichtung eine Anwesenheit eines Lebewesens in der Umgebung detektiert. Gemäß der Erfindung wird eine Desinfektionsleistung reduziert und/oder ein Warnsignal ausgegeben. Die Detektionseinrichtung des Desinfektionsroboters prüft, ob sich Menschen oder Tiere in der Umgebung des Desinfektionsroboters befinden. Die Erkennung von Lebewesen kann beispielsweise mittels einer Kamera und Objekterkennungssoftware, mittels eines LIDAR-Systems, mittels Ultraschallsensorik, mittels Radarsensorik oder ähnlichen Detektionsverfahren erfolgen. Des Weiteren kann beispielsweise auch ein Abstand eines Menschen zu dem Desinfektionsroboter geschätzt werden, beispielsweise indem die Reichweite eines Bluetoothsignals eines Mobiltelefons gemessen wird, das der Nutzer des Desinfektionsroboters bei sich trägt. Erfindungsgemäß ist vorgesehen, dass die Desinfektionsleistung reduziert wird, nämlich auf ein Maß, welches für einen Menschen oder ein Tier gesundheitlich unbedenklich ist. Gemäß einer weiteren erfindungsgemäßen Ausführung kann die Steuereinrichtung des Desinfektionsroboters ein akustisches, optisches oder anderweitig von dem Nutzer erkennbares Warnsignal abgeben, dass der Desinfektionsroboter aktuell oder in naher Zukunft eine Desinfektionstätigkeit in der Umgebung durchführt. Insbesondere kann ein solches Warnsignal auch auf einem Nutzerendgerät, beispielsweise einem Mobiltelefon oder ähnlichem, ausgegeben werden.

Insgesamt wird mit der Erfindung somit ein Desinfektionsroboter geschaffen, bei welchem zunächst ein Setup der Desinfektionsumgebung und anschließend eine Desinfektion durchgeführt wird. Zusätzlich kann später noch eine Dokumentation über erfolgreich durchgeführte oder noch ausstehende Desinfektionstätigkeiten erstellt werden. Während des Setups werden zunächst beispielsweise Räume der Umgebung innerhalb einer Umgebungskarte vermerkt. Des Weiteren werden zu desinfizierende Stellen, beispielsweise Teilbereiche oder Objekte des Raumes, als zu desinfizierend markiert. Gegebenenfalls erfolgt eine Definition, auf welche Art und Weise desinfiziert werden soll, nämlich beispielsweise mittels Desinfektionslösung, Plasma oder UV-Licht. Des Weiteren können auch eine Höhe, ein Winkel oder sonstige Zugänglichkeit des Ortes in der Umgebungskarte gespeichert werden, ebenso auch eine voraussichtliche Dauer für die auszuführende Desinfektionstätigkeit. Anschließend erfolgt die Desinfektion des Teilbereiches bzw. Objektes, nämlich bevorzugt im Rahmen eines zuvor festgelegten Zeitplans. Alternativ kann der Betrieb des Desinfektionsroboters auch manuell gestartet werden. Der Desinfektionsroboter kann vollautomatisch einen Verfahrweg zur Ausführung mehrerer Desinfektionstätigkeiten planen, wobei er anschließend zu einem ersten Desinfektionsort fährt oder fliegt, dort die Desinfektionseinrichtung auf den zu desinfizierenden Teilbereich oder das zu desinfizierende Objekt ausrichtet, nämlich beispielsweise in Bezug auf eine Höhe oder einen Winkel, und anschließend eine Desinfektion durchführt. Anschließend verfährt oder fliegt der Desinfektionsroboter zu einem nächsten definierten Desinfektionsort. Anschließend kann der Desinfektionsroboter für einen Nutzer einen Report erstellen, welcher Informationen über bereits desinfizierte oder noch zu desinfizierende Teilbereiche oder Objekte der Umgebung enthält. Der Nutzer kann beispielsweise auf einem Nutzerendgerät über die Verfügbarkeit der Dokumentation unterrichtet werden. Die Dokumentation kann anschließend wieder gelöscht oder in einem Speicher gespeichert werden.

### Kurze Beschreibung der Zeichnungen

Im Folgenden wird die Erfindung anhand von Ausführungsbeispielen näher erläutert. Es zeigen:
- Fig. 1: eine Umgebung mit einem erfindungsgemäßen Desinfektionsroboter und einem zu desinfizierenden Objekt bzw. Teilbereich,
- Fig. 2: den Desinfektionsroboter während der Ausführung einer Desinfektionstätigkeit,
- Fig. 3: ein Nutzerendgerät mit einer Umgebungskarte und darin gespeicherten zu desinfizierenden Teilbereichen und Objekten

### Beschreibung der Ausführungsformen

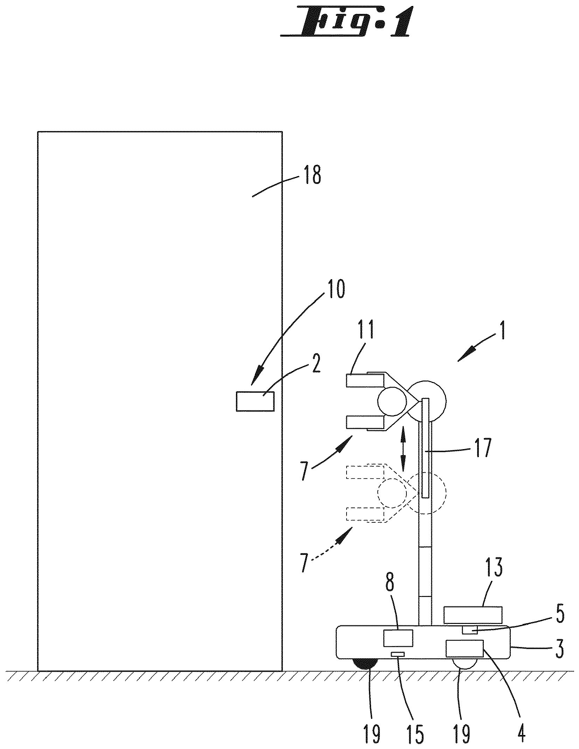

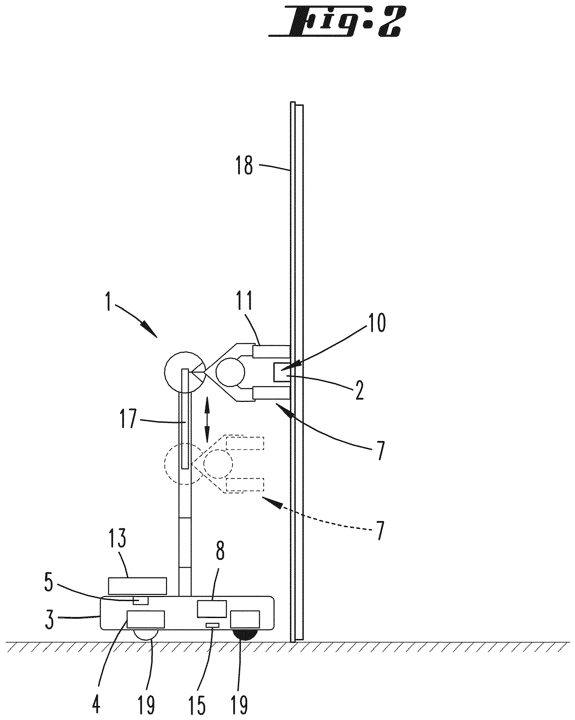

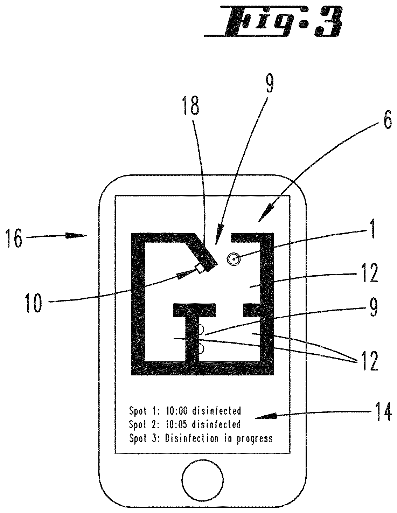

Die Fig. 1 und 2 zeigen eine Umgebung mit einem Desinfektionsroboter 1 und einem Objekt 10 mit einer zu desinfizierenden Oberfläche 2. Bei dem Objekt 10 handelt es sich hier beispielsweise um den Türgriff einer Tür 18, die mehrere Räume 12 einer Wohnung (siehe Grundriss gemäß Fig.3) voneinander trennt. Andere Objekte 10 können auch z.B. die Tür 18, Möbel, Dekorationsgegenstände, Fensterbänke oder andere sein.

Der Desinfektionsroboter 1 verfügt über ein Gerätegehäuse 3, eine Antriebseinrichtung 4, eine Navigationseinrichtung 5 und eine Desinfektionseinrichtung 7. Die Antriebseinrichtung 4 dient der Fortbewegung des Desinfektionsroboters 1 durch die Umgebung und treibt mehrere Räder 19 des Desinfektionsroboters 1 an. Die Navigationseinrichtung 5 dient der Orientierung und Selbstlokalisierung des Desinfektionsroboters 1 in der Umgebung. Der Navigationseinrichtung 5 ist eine Detektionseinrichtung 13 zugeordnet, welche zur Detektion von Objekten 10 in der Umgebung geeignet ist. Bei der Detektionseinrichtung 13 kann es sich beispielsweise um einen Abstandssensor, insbesondere Lasersensor, handeln, welcher Abstände zwischen dem Desinfektionsroboter 1 und Objekten 10 der Umgebung messen kann. Die Detektionseinrichtung 13 bzw. Navigationseinrichtung 5 wertet die detektierten Abstandswerte aus und erstellt eine Umgebungskarte 6 der Umgebung, welche vorzugsweise einen Grundriss der Räume 12 mit darin vorhandenen Objekten 10 aufweist. Diese Umgebungskarte 6 kann die Navigationseinrichtung 5 für eine Fahrtplanung des Desinfektionsroboters 1 durch die Umgebung verwenden. Insbesondere kann sich der Desinfektionsroboter 1 anhand der Umgebungskarte 6 lokalisieren und dementsprechend seine Position und Orientierung relativ zu den Objekten 10 der Umgebung bestimmen. In der Umgebungskarte 6 können neben den Objekten 10 auch Teilbereiche 9 der Umgebung definiert sein, welche Unterbereiche eines Raumes 12 der Umgebung sind, beispielsweise ein Bereich unterhalb eines Waschbeckens oder einer Toilette in einem Badezimmer, ein Eingangsbereich hinter einer Haustür oder ähnliches.

Wie dargestellt verfügt der Desinfektionsroboter 1 des Weiteren über die Desinfektionseinrichtung 7, welche gemäß unterschiedlichen Ausführungsformen eine UV-Lichtquelle, eine Plasmaquelle und/oder eine Sprüheinrichtung zum Versprühen einer Desinfektionslösung aufweisen kann. Der Desinfektionseinrichtung 7 ist eine Abschirmeinrichtung 11 zugeordnet, welche verhindert, dass UV-Strahlung, Plasma oder Desinfektionslösung außerhalb der durch die Abschirmeinrichtung 11 gebildeten Umhausung in die Umgebung freigesetzt wird. Zu diesem Zweck ist die Abschirmeinrichtung 11 nach der Art eines lediglich einseitig offenen Gehäuses ausgebildet (in den Fig. 1 und 2 geschnitten dargestellt), nämlich derart, dass die Abschirmeinrichtung 11 in Zusammenwirkung mit der Tür 18 ein vollständig geschlossenes Volumen umgibt, in welchem das zu desinfizierende Objekt 10, nämlich hier die Türklinke, eingehaust ist. Die Wandungen der Abschirmeinrichtung 11 sind bezogen auf deren wirksame Länge und deren Abstand zueinander variierbar. Die Längen und Abstände können mittels einer Verlagerungseinrichtung 17 geändert werden, welche beispielsweise einen Elektromotor und Führungen für die Verlagerungsbewegungen aufweist. Die Verlagerungseinrichtung 17 ist hier des Weiteren auch eingerichtet, eine Höhenveränderung der Desinfektionseinrichtung 7 relativ zu dem Gerätegehäuse 3 des Desinfektionsroboters 1 vorzunehmen. Auf diese Art und Weise kann die Abschirmeinrichtung 11 insgesamt in Bezug auf die Höhe und Größe des abgeschirmten Volumens an die Position und Größe des zu desinfizierenden Objektes 10 bzw. Teilbereiches 9 angepasst werden.

Der Desinfektionsroboter 1 weist zudem eine Steuereinrichtung 8 auf, welche ausgebildet ist, die Ausführung einer Desinfektionstätigkeit durch die Desinfektionseinrichtung 7 zu steuern. Der Steuereinrichtung 8 ist ein Speicher 15 zugeordnet, der hier beispielsweise einen Desinfektionsplan 14 mit zeitlich definiert im Voraus geplanten Desinfektionstätigkeiten enthält. Der Speicher 15 kann alternativ zu einem lokalen Speicher 15 auch ein virtueller Speicher sein, der sich in einer sogenannten "Cloud" befindet, das heißt auf einem entfernten Server, auf den die Steuereinrichtung 8 des Desinfektionsroboters 1 zugreifen kann, insbesondere über ein Heimkommunikationsnetzwerk, insbesondere per WLAN, oder über das Internet.

In Fig. 3 ist ein Nutzerendgerät 16 dargestellt, hier in der Form eines Mobiltelefons. Auf dem Nutzerendgerät 16 ist eine Applikation installiert, mittels welcher der Nutzer auf den Desinfektionsroboter 1 zugreifen kann, um Informationen und Steuerbefehle an den Desinfektionsroboter 1 zu übermitteln bzw. von diesem zu erhalten. Die Applikation visualisiert dem Nutzer des Nutzerendgerätes 16 einen Grundriss der von dem Desinfektionsroboter 1 erstellten Umgebungskarte 6 mit einer Mehrzahl von Räumen 12 und darin definierten Teilbereichen 9 sowie Objekten 10. In Fig. 3 ist lediglich beispielhaft nur die Klinke der Tür 18 als Objekt 10 markiert. Des Weiteren ist in der Umgebungskarte 6 eine momentane Position des Desinfektionsroboters 1 angegeben. Unterhalb der Umgebungskarte 6 ist ein Desinfektionsplan 14 dargestellt, welcher insgesamt drei Desinfektionsaufgaben für den Desinfektionsroboter 1 enthält, nämlich bezeichnet mit "Spot 1", "Spot 2" und "Spot 3". Der Desinfektionsplan 14 gibt vor, welche Teilbereiche 9 bzw. Objekte 10 der Umgebung in welcher zeitlichen Reihenfolge desinfiziert werden sollen. Hier dient der Desinfektionsplan 14 gleichzeitig als ein Protokoll über bereits erfolgreich abgeschlossene Desinfektionstätigkeiten. Erkennbar sind die Desinfektionstätigkeiten "Spot 1" und "Spot 2" bereits abgeschlossen. Eine Desinfektionstätigkeit "Spot 3" befindet sich gerade noch in der Bearbeitung.

Die Erfindung funktioniert gemäß einer ersten möglichen Ausführung so, dass der Nutzer in der durch den Desinfektionsroboter 1 erstellten Umgebungskarte 6 Teilbereiche 9 und/oder Objekte 10 markiert, welche durch die Desinfektionseinrichtung 7 des Desinfektionsroboters 1 desinfiziert werden sollen. Dabei kann der Nutzer gleichzeitig auch als Desinfektionsplan 14 die Reihenfolge für die Desinfektionstätigkeiten festlegen. Ebenso kann er Angaben bezüglich einer Art, einer Höhe, einer Größe und/oder einer Orientierung des zu desinfizierenden Teilbereiches 9 bzw. Objektes 10 machen, so dass die Verlagerungseinrichtung 17 die Desinfektionseinrichtung 7 dann entsprechend positionieren und anpassen kann. Anschließend startet der Nutzer die Desinfektionsfahrt des Desinfektionsroboters 1 über einen in der Applikation vorhandenen Startknopf (nicht dargestellt). Alternativ ist es möglich, dass der Desinfektionsroboter 1 vollautomatisch eine Desinfektionstätigkeit startet, wenn eine vordefinierte Zeit für die Desinfektion erreicht ist. Die Navigationseinrichtung 5 des Desinfektionsroboters 1 plant einen Pfad durch die Umgebung, insbesondere die in der Umgebungskarte 6 verzeichneten Räume 12, um auf optimalem, insbesondere möglichst kurzem oder energiesparendem Weg zu den zu desinfizierenden Teilbereichen 9 oder Objekten 10 zu gelangen. Hier steuert die Navigationseinrichtung 5 beispielsweise zunächst die in Fig. 1 dargestellte Tür 18 mit dem daran angeordneten zu desinfizierenden Objekt 10 (Türklinke) an. Sofern der Nutzer noch keine Information darüber hinterlassen hat, in welcher Höhe sich das Objekt 10 befindet, kann die Detektionseinrichtung 13 selbst entsprechende Informationen über das zu desinfizierende Objekt 10 ermitteln.

Sodann verlagert die Verlagerungseinrichtung 17 die Desinfektionseinrichtung 7 inklusive der Abschirmeinrichtung 11 gemäß Fig. 2 so, dass das Objekt 10 von der Abschirmeinrichtung 11 umfangen ist. Hier wird die Abvschirmeinrichtung 11 an die Tür 18 angelegt. Dabei bilden die Tür 18 und die Wandungen der Abschirmeinrichtung 11 ein geschlossenes Gehäuse, in welchem sich das Objekt 10 befindet. Anschließend wird eine Desinfektionsquelle der Desinfektionseinrichtung 7 gestartet. Die Desinfektionsquelle kann beispielsweise eine UV-Lampe, eine Plasmaquelle oder auch eine Sprüheinrichtung zum Versprühen einer Desinfektionslösung sein. Die Abschirmeinrichtung 11 sorgt während der Ausführung der Desinfektion dafür, dass keine Strahlung, Plasma oder Sprühlösung aus der Abschirmeinrichtung 11 raus in die Umgebung gelangt. Sobald die Desinfektion erfolgreich abgeschlossen ist, übermittelt die Steuereinrichtung 8 eine Information über die Beendigung der Desinfektionstätigkeit an den Speicher 15, welcher diese Information an die Applikation des Nutzerendgerätes 16 weiterleitet. Sodann wird in dem Desinfektionsplan 14 festgehalten, dass die Desinfektionstätigkeit beendet ist. Der Desinfektionsplan 14 dient somit gleichzeitig als Protokoll für die bereits erfolgreich abgeschlossenen Desinfektionstätigkeiten.

Zusätzlich kann vorgesehen sein, dass die Detektionseinrichtung 13 des Desinfektionsroboters 1 die Umgebung überwacht und detektiert, wenn ein Mensch oder ein Tier den Raum 12, in welchem der Desinfektionsroboter 1 tätig ist, betritt. Die Detektion des Menschen bzw. des Tieres kann mittels einer Auswertung eines Kamerabildes erfolgen, oder alternativ anhand der Erkennung des Menschen bzw. Tieres als sich schnell bewegendes "Objekt". Sofern die Anwesenheit eines Lebewesens erkannt wird, kann der Desinfektionsroboter 1 beispielsweise ein Warnsignal ausgeben, das zumindest einen Menschen darüber informiert, dass die Desinfektionseinrichtung 7 derzeit aktiv ist bzw. in Kürze aktiviert wird. Alternativ kann die Steuereinrichtung 8 in dem Fall jedoch auch vollautomatisch eine Desinfektionstätigkeit abbrechen oder aufschieben. Ebenso kann auch vorgesehen sein, dass die Steuereinrichtung 8 die Desinfektionsleistung für die momentane Desinfektionstätigkeit reduziert, so dass eine für einen Menschen oder ein Tier unschädliche Dosis abgegeben wird.

Gemäß einer alternativen Verfahrensführung kann vorgesehen sein, dass der Desinfektionsroboter 1 zu desinfizierende Teilbereiche 9 oder zu desinfizierende Objekte 10 mittels seiner Detektionseinrichtung 13 automatisch erkennt. Dies kann beispielsweise durch eine Probennahme und Untersuchung der Probe in Bezug auf Viren, Bakterien, Pilze oder ähnliche gesundheitsgefährdende Stoffe geschehen. Sofern dann feststellt wird, dass das entsprechende Objekt 10 oder der korrespondierende Teilbereich 9 desinfiziert werden sollte, kann die Steuereinrichtung 8 einen Desinfektionsbefehl an die Desinfektionseinrichtung 7 übermitteln, um eine Desinfektionstätigkeit vollautomatisch zu starten. Alternativ kann auch vorgesehen sein, dass der Nutzer zunächst eine Nachricht auf sein Nutzerendgerät 16 bekommt und dann entscheiden kann, ob das Objekt 10 bzw. der Teilbereich 9 tatsächlich desinfiziert werden soll. Gegebenenfalls kann der Nutzer auch selbst die Art der Desinfektion wählen, sofern der Desinfektionsroboter 1 über verschiedene Desinfektionseinrichtungen 7 verfügt, die wahlweise eine Desinfektion mittels UV-Licht, Plasma oder Desinfektionslösung ermöglichen.

Obwohl die Ausführungsformen vorstehend anhand eines bodengestützten Desinfektionsroboters 1 erläutert wurden, kann es sich bei dem Desinfektionsroboter 1 alternativ auch um eine Flugdrohne handeln, welche Objekte 10 bzw. Teilbereiche 9 anfliegen kann und dann eine Desinfektion auch insbesondere von oben herab durchführen kann. Dies empfiehlt sich ganz besonders, wenn beispielsweise eine Desinfektionslösung von oben auf Objekte 10 bzw. Teilbereiche 9 aufgesprüht werden soll.

### Liste der Bezugszeichen

- 1: Desinfektionsroboter
- 2: Oberfläche
- 3: Gerätegehäuse
- 4: Antriebseinrichtung
- 5: Navigationseinrichtung
- 6: Umgebungskarte
- 7: Desinfektionseinrichtung
- 8: Steuereinrichtung
- 9: Teilbereich
- 10: Objekt
- 11: Abschirmeinrichtung
- 12: Raum
- 13: Detektionseinrichtung
- 14: Desinfektionsplan
- 15: Speicher
- 16: Nutzerendgerät
- 17: Verlagerungseinrichtung
- 18: Tür
- 19: Rad

## Patentansprüche

1. Sich selbsttätig fortbewegender Desinfektionsroboter (1) zum Desinfizieren von Oberflächen (2), mit einem Gerätegehäuse (3), einer Antriebseinrichtung (4) zur Fortbewegung des Desinfektionsroboters (1) innerhalb einer Umgebung mit mindestens einem Raum (12), einer Desinfektionseinrichtung (7) zum Ausführen einer Desinfektionstätigkeit innerhalb der Umgebung und einer Steuereinrichtung (8) zum Steuern der Desinfektionstätigkeit, wobei die Steuereinrichtung (8) eingerichtet ist, vollautomatisiert eine lokal begrenzte Desinfektion in ausschließlich nur einem dafür vordefinierten Teilbereich (9) des Raumes (12) der Umgebung oder eine lokal begrenzte Desinfektion von ausschließlich nur einem dafür definierten Objekt (10) in dem Raum (12) der Umgebung zu steuern, wobei die Desinfektionseinrichtung (7) zum Erreichen einer lokal begrenzten Desinfektionswirkung eine Abschirmeinrichtung (11), nämlich Einhausung, zum Separieren des zu desinfizierenden, lokal begrenzten Teilbereiches (9) von benachbarten Teilbereichen (9) desselben Raumes (12) aufweist, wobei die Abschirmeinrichtung (11) ein lediglich einseitig offenes Gehäuse ist, welches um das zu desinfizierende Objekt (10) oder an die zu desinfizierende Oberfläche (2) gelegt wird, so dass die Desinfektion lediglich innerhalb des Gehäuses stattfindet, jedoch nicht außerhalb des Gehäuses, **dadurch gekennzeichnet, dass** der Desinfektionsroboter (1) eine Navigationseinrichtung (5) zur Navigation und Selbstlokalisierung des Desinfektionsroboters (1) innerhalb der Umgebung anhand einer Umgebungskarte (6) aufweist, und wobei die Steuereinrichtung (8) eingerichtet ist, auf einen Desinfektionsplan (14) zuzugreifen, welcher in einem Speicher (15) des Desinfektionsroboters (1)
oder einem in Datenkommunikation mit dem Desinfektionsroboter (1) stehenden externen Speicher (15) gespeichert ist, wobei der Desinfektionsplan (14) eine Vorgabe über eine Desinfektionsleistung, welche die Desinfektionswirkung festlegt, während einer Desinfektionstätigkeit aufweist, wobei stärker kontaminierte Teilbereiche (9) oder Objekte (10) der Umgebung stärker desinfiziert werden als demgegenüber lediglich leicht kontaminierte Objekte (10) oder Teilbereiche (9), und wobei der Desinfektionsroboter (1) eine Detektionseinrichtung (13) aufweist, welche eingerichtet ist, Lebewesen in der Umgebung zu erkennen, wobei die Steuereinrichtung (8) eingerichtet ist, eine Desinfektionsleistung zu reduzieren und/oder ein Warnsignal auszugeben, wenn die Detektionseinrichtung (13) eine Anwesenheit eines Lebewesens in der Umgebung detektiert.

2. Desinfektionsroboter (1) nach Anspruch 1, **dadurch gekennzeichnet, dass** der zu desinfizierende Teilbereich (9) der Umgebung ein in der Umgebungskarte (6) gespeicherter Ortspunkt, eine in der Umgebungskarte (6) gespeicherte Teilfläche oder ein in der Umgebungskarte (6) gespeichertes Teilvolumen ist.

3. Desinfektionsroboter (1) nach einem der vorhergehenden Ansprüche, **gekennzeichnet durch** eine Kommunikationsschnittstelle zum Vordefinieren des zu desinfizierenden Teilbereiches (9) oder des zu desinfizierenden Objektes (10) durch einen Nutzer oder Hersteller des Desinfektionsroboters (1), und/oder **gekennzeichnet durch** eine Detektionseinrichtung (13) zum automatischen Erkennen des zu desinfizierenden Teilbereiches (9) oder des zu desinfizierenden Objektes (10) in der Umgebung.

4. Desinfektionsroboter (1) nach einem der vorhergehenden Ansprüche,
**dadurch gekennzeichnet, dass** die Steuereinrichtung (8) eingerichtet ist, auf einen Desinfektionsplan (14) zuzugreifen, welcher in einem Speicher (15) des Desinfektionsroboters (1) oder einem in Datenkommunikation mit dem Desinfektionsroboter (1) stehenden externen Speicher (15) gespeichert ist, wobei der Desinfektionsplan (14) eine Vorgabe über eine Reihenfolge von mehreren auszuführenden Desinfektionstätigkeiten, eine Vorgabe über eine Wiederholungsfrequenz von Desinfektionstätigkeiten und/oder eine Vorgabe über einen Desinfektionszeitpunkt einer Desinfektionstätigkeit aufweist.

5. Desinfektionsroboter (1) nach Anspruch 4, **dadurch gekennzeichnet, dass** der Desinfektionsplan (14) durch einen Nutzer des Desinfektionsroboters (1) variierbar ist, insbesondere mit Hilfe einer auf einem externen Nutzerendgerät (16) gespeicherten Applikation, welche eingerichtet ist, auf den Speicher (15) zuzugreifen.

6. Desinfektionsroboter (1) nach einem der vorhergehenden Ansprüche, **dadurch gekennzeichnet, dass** die Desinfektionseinrichtung (7) eine UV-Lichtquelle, insbesondere mindestens eine UV-LED, eine Plasmaquelle und/oder eine Sprüheinrichtung zum Versprühen einer Desinfektionslösung aufweist.

7. Desinfektionsroboter (1) nach einem der vorhergehenden Ansprüche, **gekennzeichnet durch** eine Verlagerungseinrichtung (17) zur Verlagerung, insbesondere Höhenveränderung, der Desinfektionseinrichtung (7) relativ zu dem Gerätegehäuse (3).

8. Desinfektionsroboter (1) nach einem der vorhergehenden Ansprüche, **dadurch gekennzeichnet, dass** der Desinfektionsroboter (1) eine Flugdrohne oder ein Bodenfahrzeug ist.

9. Desinfektionsroboter (1) nach einem der vorhergehenden Ansprüche, **gekennzeichnet durch** eine Detektionseinrichtung (13), welche eingerichtet ist, Lebewesen, insbesondere Menschen und Tiere, in der Umgebung zu erkennen, wobei die Steuereinrichtung (8) des Desinfektionsroboters (1) eingerichtet ist, eine Desinfektionstätigkeit zeitlich aufzuschieben und/oder eine Desinfektionstätigkeit abzubrechen, wenn die Detektionseinrichtung (13) eine Anwesenheit eines Lebewesens in der Umgebung detektiert.

## Claims

1. An automatically moving disinfection robot (1) for disinfecting surfaces (2), comprising a device housing (3), a drive means (4) for the movement of the disinfection robot (1) within a surrounding area comprising at least one room (12), a disinfection means (7) for performing a disinfection activity within the surrounding area, and a control means (8) for controlling the disinfection activity, wherein the control means (8) is configured to control a locally limited disinfection in only one subregion (9) of the room (12) of the surrounding area, which is predefined for this purpose, or a locally limited disinfection of only one object (10), which is defined for this purpose, in the room (12) of the surrounding area in a fully automated manner, wherein for attaining a locally limited disinfection effect, the disinfection means (7) has a shielding means (11), namely enclosure, for separating the locally limited subregion (9) to be disinfected from adjacent subregions (9) of the same room (12), wherein the shielding means (11) is a housing, which is open only on one side and which is placed around the object (10) to be disinfected or against the surface (2) to be disinfected, so that the disinfection only takes place within the housing, but not outside of the housing, **characterized in that** the disinfection robot (1) has a navigation device (5) for navigation and self-localization of the disinfection robot (1) within the environment on the basis of an environment map (6), and wherein the control device (8) is arranged to access a disinfection plan (14) stored in a memory (15) of the disinfection robot (1) or an external memory (15) which is in data communication with the disinfection robot (1), the disinfection plan (14) having a specification of a disinfection performance, which specifies the disinfection effect, during a disinfection activity, wherein more heavily contaminated sub-areas (9) or objects (10) of the environment are disinfected to a greater extent than, in contrast, only lightly contaminated objects (10) or sub-areas (9), and wherein the disinfection robot (1) has a detection device (13) which is set up to detect living beings in the environment, the control device (8) being set up to reduce a disinfection output and/or to emit a warning signal if the detection device (13) detects the presence of a living being in the environment.

2. The disinfection robot (1) according to claim 1, **characterized in that** the subregion (9) of the surrounding area to be disinfected is a location point stored in the surrounding area map (6), a subsurface stored in the surrounding area map (6), or a partial volume stored in the surrounding area map (6).

3. The disinfection robot (1) according to one of the preceding claims, **characterized by** a communication interface for predefining the subregion (9) to be disinfected or the object (10) to be disinfected by a user or manufacturer of the disinfection robot (1), and/or **characterized by** a detection means (13) for automatically recognizing the subregion (9) to be disinfected or the object (10) to be disinfected in the surrounding area (9).

4. The disinfection robot (1) according to one of the preceding claims, **characterized in that** the control means (8) is configured to access a disinfection plan (14), which is stored in a memory (15) of the disinfection robot (1) or an external memory (15), which is in data communication with the disinfection robot (1), wherein the disinfection plan (14) has a specification relating to an order of several disinfection activities to be performed, a specification relating to a repetition frequency of disinfection activities and/or a specification relating to a disinfection point in time of a disinfection activity.

5. The disinfection robot (1) according to claim 4, **characterized in that** the disinfection plan (14) can be varied by a user of the disinfection robot (1), in particular with the help of an application, which is stored on an external user terminal (16) and which is configured to access the memory (15).

6. The disinfection robot (1) according to one of the preceding claims, **characterized in that** the disinfection means (7) has a UV light source, in particular at least one UV LED, a plasma source, and/or a spraying means for spraying a disinfection solution.

7. The disinfection robot (1) according to one of the preceding claims, **characterized by** a displacement means (17) for displacing, in particular height-changing, the disinfection means (7) relative to the device housing (3).

8. The disinfection robot (1) according to one of the preceding claims, **characterized in that** the disinfection robot (1) is an aerial drone or a ground vehicle.

9. The Disinfection robot (1) according to one of the preceding claims, **characterized by** a detection means (13), which is configured to recognize living beings, in particular humans and animals, in the surrounding area, wherein the control means (8) of the disinfection robot (1) is configured to temporally postpone a disinfection activity and/or to terminate a disinfection activity, when the detection means (13) detects a presence of a living being in the surrounding area.

## Revendications

1. Robot de désinfection (1) se déplaçant automatiquement pour désinfecter des surfaces (2), avec un boîtier d'appareil (3), un dispositif d'entraînement (4) pour le déplacement du robot de désinfection (1) à l'intérieur d'un environnement avec au moins un espace (12), un dispositif de désinfection (7) pour exécuter une activité de désinfection à l'intérieur de l'environnement et un dispositif de commande (8) pour commander l'activité de désinfection, le dispositif de commande (8) étant conçu pour commander de manière entièrement automatisée une désinfection limitée localement dans exclusivement une seule zone partielle (9) prédéfinie à cet espace (12) de l'environnement ou une désinfection limitée localement exclusivement d'un seul objet (10) défini à cet espace (12) de l'environnement, le dispositif de désinfection (7) présentant, pour obtenir un effet de désinfection limité localement, un dispositif de blindage (11), à savoir une enceinte, pour séparer la zone partielle (9) limitée localement à des zones partielles (9) voisines de la même espace (12), le dispositif de blindage (11) étant un boîtier ouvert uniquement d'un côté, qui est placé autour de l'objet à désinfecter (10) ou contre la surface à désinfecter (2), de sorte que la désinfection a lieu uniquement à l'intérieur du boîtier, mais pas à l'extérieur du boîtier, **caractérisé en ce que** le robot de désinfection (1) comprend un dispositif de navigation (5) pour la navigation et l'autolocalisation du robot de désinfection (1) à l'intérieur d'un environnement à l'aide d'une mémoire externe (15), et le dispositive de commande (8) étant conçu pour accéder à un plan de désinfection (14) qui est stocké dans une mémoire de le robot de désinfection (1) ou dans une mémoire extérieur en communication de données avec le robot de désinfection (1), le présentant une prescription concernant une puissance de désinfection, qui détermine l'effet de désinfection, pendant une activité de désinfection, des zones partielles (9) ou des objets (10) de l'environnement plus fortement contaminés étant désinfectés plus fortement qu'en revanche des objets (10) ou des zones partielles (9) seulement légèrement contaminés, et le robot de désinfection (1) présente un dispositif de détection (13) qui est conçu pour reconnaître des êtres vivants dans l'environnement, le dispositif de commande (8) étant conçu pour réduire une prestation de désinfection et/ ou pour émettre un signal d'avertissement lorsque le dispositif de détection (13) détecte la présence d'un être vivant dans l'environnement.

2. Robot de désinfection (1) selon la revendication 1, **caractérisé en ce que** la zone partielle (9) de l'environnement à désinfecter est un point local enregistré dans la carte de l'environnement (6), une surface partielle enregistrée dans la carte de l'environnement (6) ou un volume partiel enregistré dans la carte de l'environnement (6).

3. Robot de désinfection (1) selon l'une des revendications précédentes, **caractérisé par** une interface de communication pour prédéfinir la zone partielle (9) à désinfecter ou l'objet (10) à désinfecter par un utilisateur ou un fabricant du robot de désinfection (1), et/ou **caractérisé par** un dispositif de détection (13) pour reconnaître automatiquement la zone partielle (9) à désinfecter ou l'objet (10) à désinfecter dans l'environnement.

4. Robot de désinfection (1) selon l'une des revendications précédentes, **caractérisé en ce que** le dispositif de commande (8) est conçu pour accéder à un plan de désinfection (14) qui est stocké dans une mémoire (15) du robot de désinfection (1) ou dans une mémoire externe (15) en communication de données avec le robot de désinfection (1), dans lequel le plan de désinfection (14) présente une prescription concernant un ordre de plusieurs activités de désinfection à exécuter, une prescription concernant une fréquence de répétition d'activités de désinfection et/ou une prescription concernant un moment de désinfection d'une activité de désinfection.

5. Robot de désinfection (1) selon la revendication 4, **caractérisé en ce que** le plan de désinfection (14) est exploitable par un utilisateur du robot de désinfection (1), notamment à l'aide d'une application stockée sur un terminal utilisateur externe (16) et agencée pour accéder à la mémoire (15).

6. Robot de désinfection (1) selon l'une des revendications précédentes, **caractérisé en ce que** le dispositif de désinfection (7) comprend une source de lumière UV, en particulier au moins une LED UV, une source de plasma et/ou un dispositif de pulvérisation pour pulvériser une solution de désinfection.

7. Robot de désinfection (1) selon l'une des revendications précédentes, **caractérisé par** un dispositif de déplacement (17) pour le déplacement, en particulier le changement de hauteur, du dispositif de désinfection (7) par rapport au boîtier de l'appareil (3).

8. Robot de désinfection (1) selon l'une des revendications précédentes, **caractérisé en ce que** le robot de désinfection (1) est un drone volant ou un véhicule terrestre.

9. Robot de désinfection (1) selon l'une des revendications précédentes, **caractérisé par** un dispositif de détection (13) qui est conçu pour reconnaître des êtres vivants, en particulier des êtres humains et des animaux, dans l'environnement, le dispositif de commande (8) du robot de désinfection (1) étant conçu pour différer dans le temps une activité de désinfection et/ ou pour interrompre une activité de désinfection lorsque le dispositif de détection (13) détecte la présence d'un être vivant dans l'environnement.
